(19) Europäisches Patentamt
European Patent Office
Office européen des brevets

(11) Publication number: 0 231 053
A1

(12) # EUROPEAN PATENT APPLICATION

(21) Application number: 87300027.7

(22) Date of filing: 05.01.87

(51) Int. Cl.³: C 25 B 3/02
//C07C49/83

(30) Priority: 06.01.86 US 816501
06.01.86 US 816502

(43) Date of publication of application:
05.08.87 Bulletin 87/32

(84) Designated Contracting States:
BE CH DE FR GB IT LI NL

(71) Applicant: THE DOW CHEMICAL COMPANY
2030 Dow Center Abbott Road P.O. Box 1967
Midland, MI 48640(US)

(72) Inventor: Lysenko, Zenon
214 West Meadowbrook Drive
Midland Michiga 48640(US)

(72) Inventor: Bancroft, Eric E.
3008 Darby Street
Midland Michigan 48640(US)

(74) Representative: Raynor, John et al,
W.H. Beck, Greener & Co 7 Stone Buildings Lincoln's Inn
London WC2A 3SZ(GB)

(54) **Electrocatalytic method for producing quinone methides and dihydroxybenzophenones.**

(57) A process for producing dihydroxybenzophenones and quinone methides by the electrocatalytic oxidation of bis (4-hydroxyphenyl)methanes, or the quinone methide intermediate, with 2,3-dichloro-5,6-dicyano-1,4-benzoquinone (DDQ) is described. Spent oxidant, in the form of $DDQH_2$, may be recycled and electrochemically regenerated to active DDQ oxidant.

EP 0 231 053 A1

# ELECTROCATALYTIC METHOD FOR PRODUCING QUINONE METHIDES AND DIHYDROXYBENZOPHENONES

The present invention concerns a process for preparing quinone methides and dihydroxybenzophenones, and more particularly a process for electrocatalytically oxidizing bis(4-hydroxyphenyl)-methanes with 2,3-dichloro-5,6-dicyano-1,4-benzoquinone (DDQ) to produce hydroxyphenyl quinone methides and 4,4'-dihydroxybenzophenones.

Quinone methides are known to be useful as antioxidants as taught by U.S. Patent 2,940,988, which discloses the oxidation of dihydroxydiphenylmethane with lead dioxide or lead tetraacetate to produce a free radical which is subsequently reduced to quinone methide. U.S. Patent 4,032,547 discloses an oxidation process for preparing quinone alkides from the corresponding trialkyl or phenyl hindered phenols. The oxidizing agent is ferricyanide as the secondary oxidant in combination with persulfate as the primary oxidant.

Quinone methides are also useful starting materials in the preparation of dihydroxybenzophenones. The dihydroxybenzophenones may, in turn, be used as light stabilizing agents and precursors for epoxy resins, polycarbonate resins, and other thermoplastics. See, for example, U.S. Patent 3,291,837 wherein 2-hydroxy-3-halopropyl benzophenone ether stabilizing agents are prepared by the catalyzed reaction of a benzophenone intermediate with an epihalohydrin.

Other forms of benzophenones, particularly bisphenol K (4,4'-dihydroxybenzophenone) and substituted bisphenol K, are useful as monomers in the production of synthetic resins. For example, epoxy resins, which are useful as coatings, adhesives and casting resins, can be prepared from the reaction of a bisphenol and epichlorohydrin. Likewise, polycarbonate resins, which are useful as coatings and casting resins, can be prepared from the reaction of a bisphenol and phosgene.

While prior art methods for preparing quinone methides and dihydroxybenzophenones exist, to date those methods have not found significant commercial utility because of their cost, inefficiencies, or other drawbacks. Accordingly, the need exists for a process by which large quantities of quinone methides and dihydroxybenzophenones can be produced economically and at high yields.

The present invention provides a process for producing a dihydroxybenzophenone of the formula

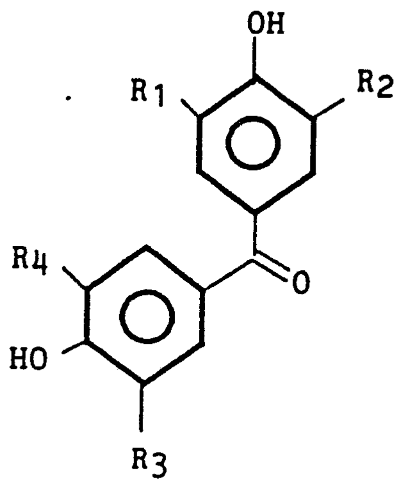

(I)

wherein each $R_1$, $R_2$, $R_3$ and $R_4$ are independently hydrogen, a straight or branched chain $C_1$-$C_{12}$ alkyl moiety, a $C_3$-$C_7$ cyclic alkyl group, halogen, a halo-($C_1$-$C_{12}$ alkyl) moiety, hydroxy, or methoxy group, which comprises electrocatalytically oxidizing a bis(4-hydroxyphenyl)methane of the formula

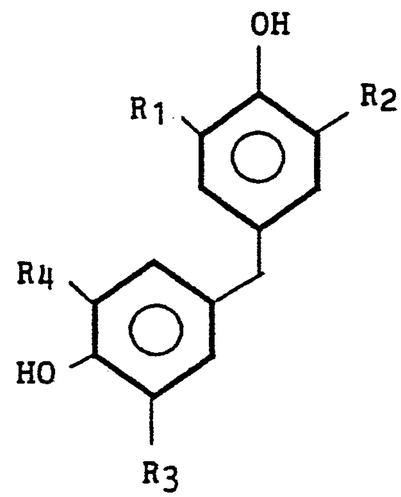

(II)

wherein $R_1$, $R_2$, $R_3$ and $R_4$ are defined as above, with 2,3-dichloro-5,6-dicyano-1,4-benzoquinone of the formula

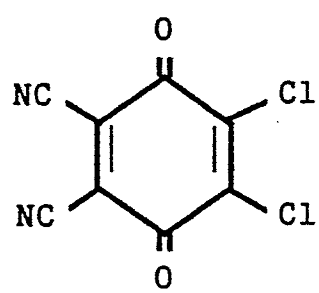

(III)

to produce a quinone methide of the formula

0231053

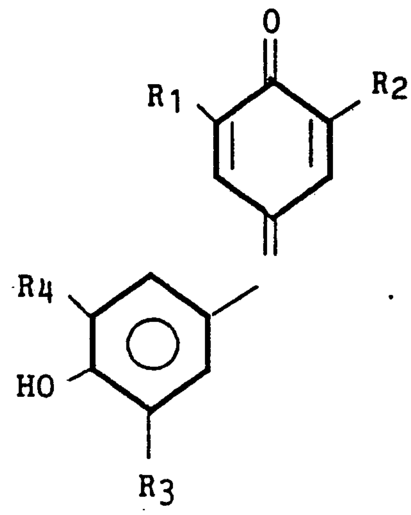

(IV)

wherein $R_1$, $R_2$, $R_3$, and $R_4$ are defined as above, which is further oxidized to provide the compounds of formula (I).

It has been found that the reaction proceeds through a quinone methide intermediate of the formula

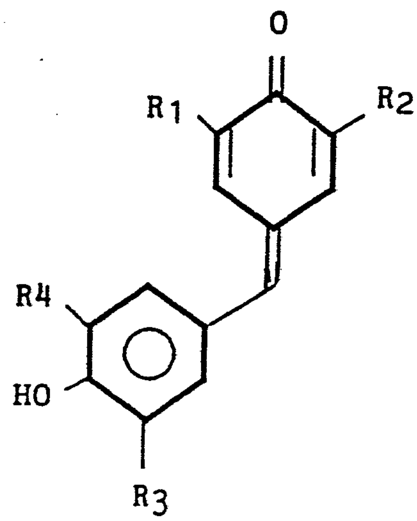

(IV)

wherein $R_1$, $R_2$, $R_3$ and $R_4$ are defined as in formula (I). Thus, it it possible to produce dihydroxybenzophenones of formula (I) directly from the quinone methide intermediates of formula (IV).

The oxidant used to oxidize the bis(4-hydroxyphenyl)methanes of formula (II) to the dihydroxybenzophenones of formula (I) is 2,3-dichloro-5,6-dicyano-1,4-benzoquinone (DDQ). It has the formula

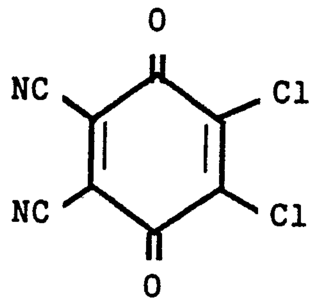

(III)

The various terms for $R_1$, $R_2$, $R_3$ and $R_4$ above are well known. Straight and branched chain $C_1$-$C_{12}$ alkyl moieties include, for example, methyl, ethyl, n-propyl, isopropyl, n-butyl, sec-butyl, tert-butyl, iso--butyl, n-pentyl, isohexyl, sec-heptyl, n-octyl, n-nonyl, sec-decyl, n-undecyl, n-dodecyl, and others. The $C_3$-$C_7$ cyclic alkyl groups include, for example, cyclopropyl, cyclobutyl, cyclopentyl, cyclo- -hexyl and cycloheptyl. Halogen includes fluorine, chlorine and bromine. Halo($C_1$-$C_{12}$ alkyl) includes, for example, trifluormethyl, 1,1-di-bromoethyl, 1,2-dichloroethyl, 1-chloro-3-bromopropyl, 1,1-difluoroethyl and others.

Preferably, the process is an electrocatalytic one wherein the DDQ oxidant is used in a homogeneous solution in the anode compartment of an electrochemical cell, which may contain for example a platinum anode. The potential applied may vary between 0.65 and 1.4 V vs. SCE and more preferably between 0.75 and 1.2 V vs. SCE and most preferably from 0.75 to 0.95 V vs. SCE.

Under these conditions, the DDQ may be used in catalytic (i.e. less than stoichiometric) amounts, i.e. between 10 and 100 mole percent relative to the starting material. Likewise, since $DDQH_2$ (below) is electrolytically oxidizable to DDQ it is possible to use $DDQH_2$ as the starting oxidant material. Since $DDQH_2$ is the form of the spent DDQ oxidant, it may be recycled and regenerated <u>in situ</u> in the process. It is not necessary to isolate either DDQ or $DDQH_2$ from the solution. $DDQH_2$ has the formula

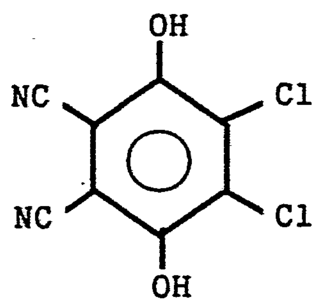

(V)

As such, the overall electrocatalytic oxidation process can be expressed as follows:

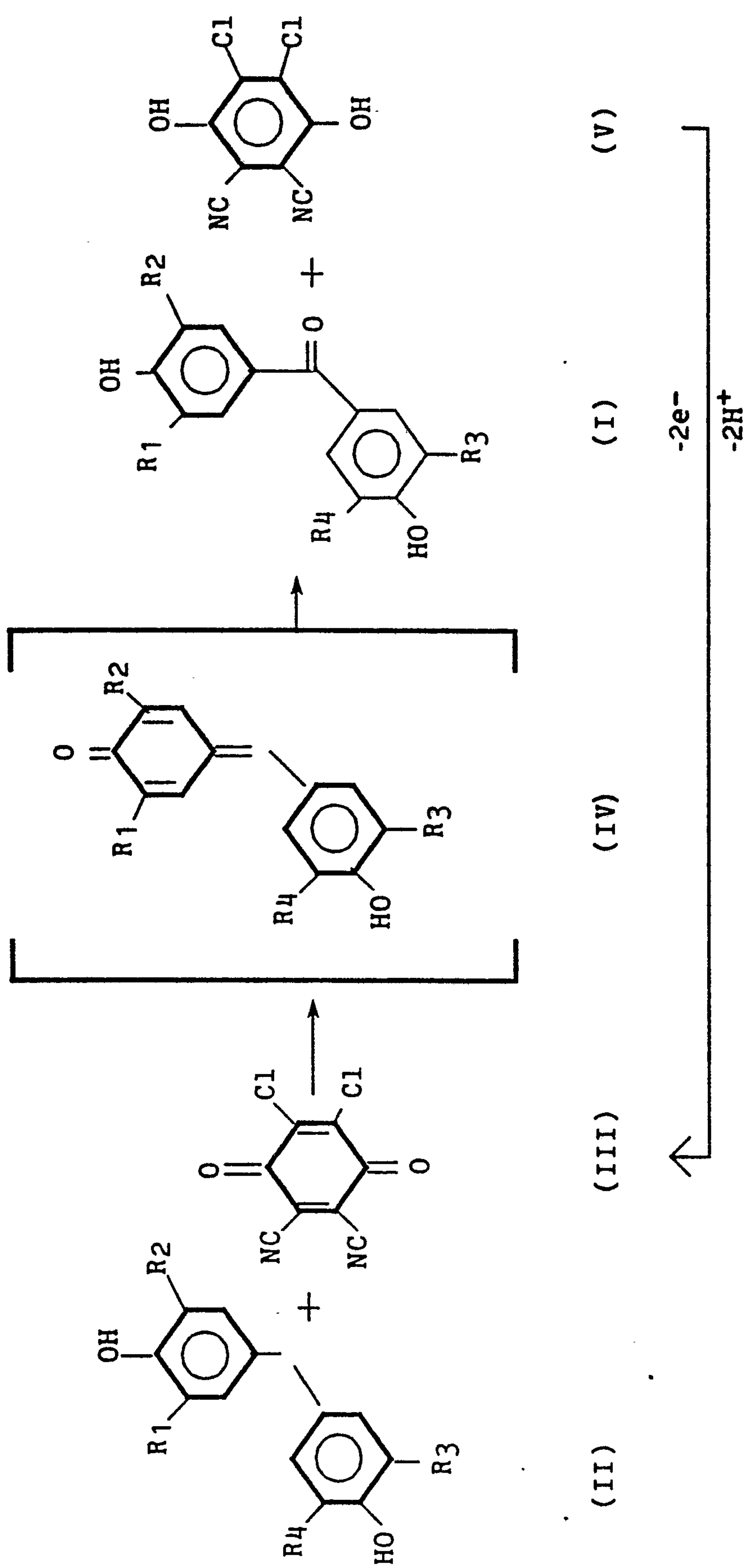


(II) (III) (IV) (I) (V)

The conversion of the bis(4-hydroxyphenyl)methane of formula (II) to the dihydroxybenzophenone of formula (I) is accomplished by the DDQ of formula (III) acting as an oxidant in homogeneous solution with the bis(4-hydroxyphenyl)methane of formula (II). The spent oxidant, $DDQH_2$ of formula (V) may, then, be recycled and regenerated to DDQ (formula III) by electro-oxidation at an electrode potential which is insufficient to directly oxidize the bis(4-hydroxyphenyl)-methane of formula (II).

With some bis(4-hydroxyphenyl)methanes, such as the tetramethyl variety, it has been found that the reaction proceeds through the quinone methide intermediate of formula (IV). In other instances, such as when the starting material is 4,4'-dihydroxydiphenyl methane, the intermediate, if any, is transient. In that instance, the overall reaction is better expressed as follows:

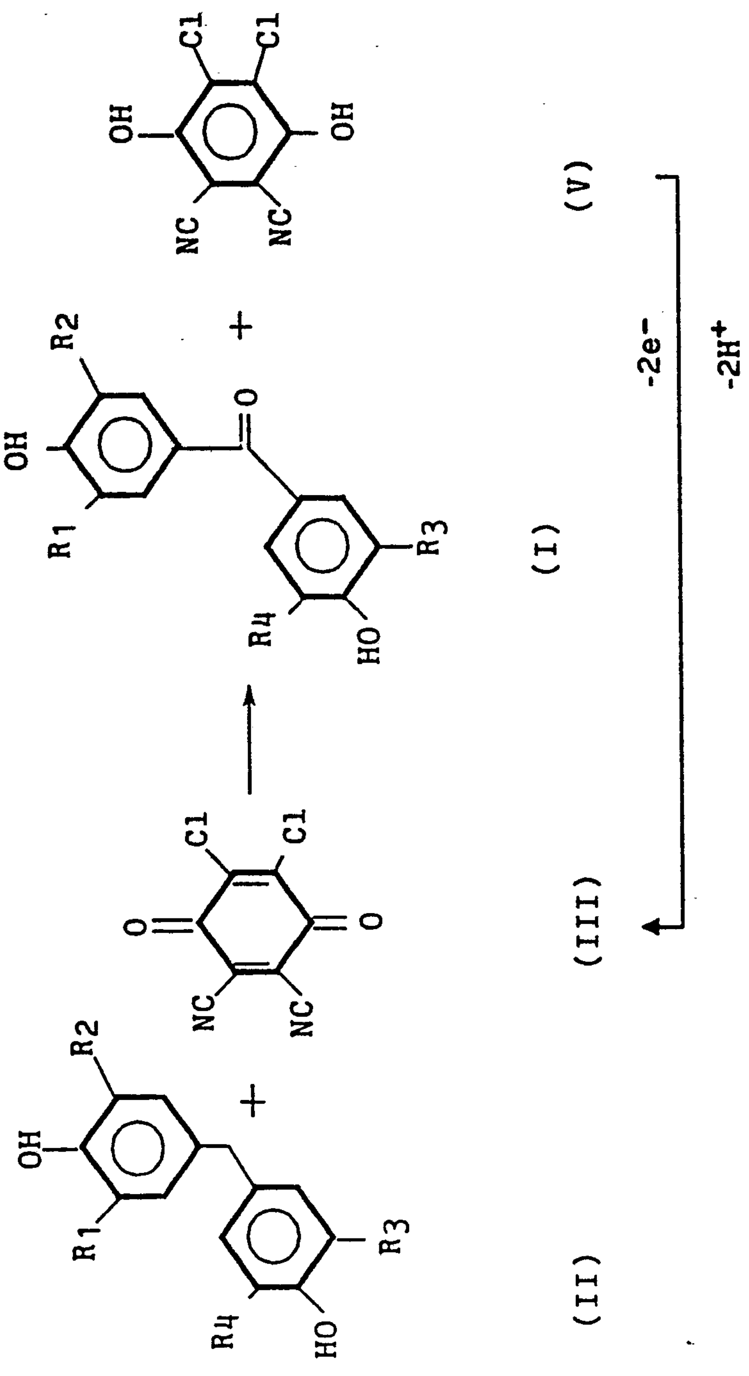


(II) (III) (I) (V)

When a quinone methide intermediate is used as the starting material, then the conversion to the dihydroxybenzophenone can be expressed as follows:

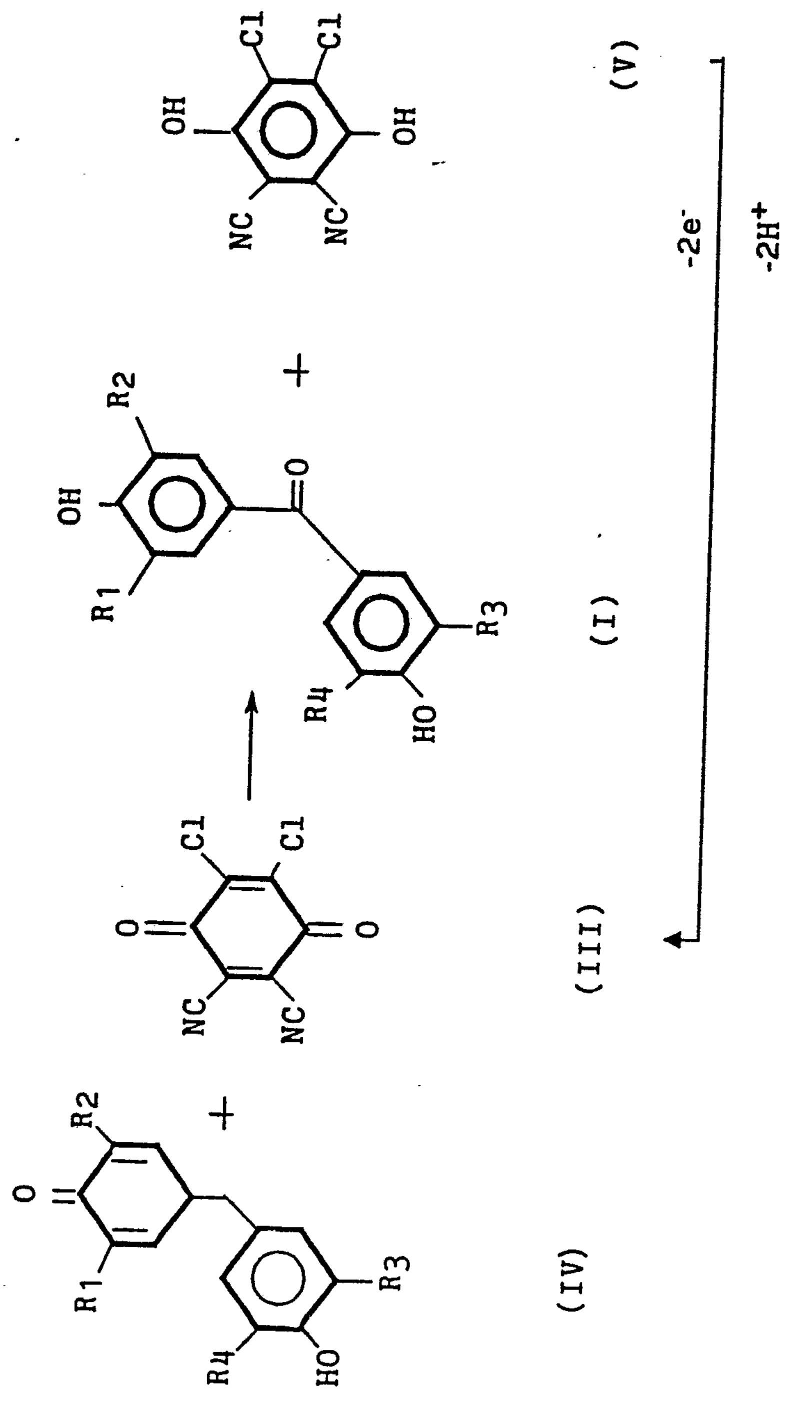


(IV) (III) (I) (V)

In any event, the result is an efficient, economical, high yield process for the production of the dihydroxybenzophenones of formula (I) which find utility as light stabilizing agents and precursors for the production of epoxy resins, polycarbonate resins, and other thermoplastics.

Accordingly, the present invention provides an inexpensive means to produce large quantities of dihydroxybenzophenones of formula (I).

The preferred process is a catalytic oxidation reaction carried out in an electrochemical cell at room temperature and pressure. A divided batch electrochemical cell is fitted with working and auxiliary electrodes and a suitable reference electrode such as a saturated calomel reference electrode (SCE). The cathode (auxiliary) compartment is filled with a supporting electrolyte solution. Any number of solvent/supporting electrolyte solutions can be used so long as they provide acceptable solubilities for bis(4-hydroxyphenyl)methanes, quinone methides, DDQ, $DDQH_2$, and the dihydroxybenzophenone.

The working electrode is the anode, which may be platinum, carbon or any other inert electrode material which remains stable at the oxidation potential. The anode compartment is filled with the supporting electrolyte solution and the starting materials. The required starting materials include both the oxidation catalyst and the substrate material. The working electrode is then biased to, and maintained at, a constant voltage vs. SCE using a three electrode potentiostat. During electrolysis, the anolyte

solution is rapidly stirred using conventional stirring equipment.

In one embodiment, the starting oxidation catalyst placed in the anode compartment is the DDQ of formula (III). DDQ is a known oxidant as taught by U.S.Patents 4,518,535; 4,056,539 and 3,102,124. It may be purchased from Aldrich Chemical Company. In another embodiment, the starting oxidation catalyst may be the $DDQH_2$ of formula (V). In that instance, the electrolytic oxidation, at an electrode potential in the range of 0.65 to 1.4 V vs. SCE, oxidizes the $DDQH_2$ to DDQ which, then serves as the oxidation catalyst. $DDQH_2$ is available as the reduced form of DDQ. Since $DDQH_2$ is produced by the reaction process, it is thus possible to recycle it in situ in the anode compartment. In addition, since the DDQ oxidant is regenerated in situ it may be used in non-stoichiometric quantities in the range of 10 to 100 mole percent relative to the starting substrate material and preferably on the order of 10 mole percent. As a result, a relatively inexpensive source of oxidant is utilized in the process. In addition, use of an electrocatalytic process offers energy saving advantages over an electrolytic process, for example.

In one embodiment, the starting substrate material placed in the anode compartment is the bis(4-hydroxyphenyl)methane of formula (II). Some of the bis(4-hydroxyphenyl)methanes of formula (II) where each $R_1$, $R_2$, $R_3$ and $R_4$ are independently hydrogen, straight or branched chain $C_1$-$C_{12}$ alkyl moieties, $C_3$-$C_7$ cyclic alkyl groups, halogen, halo($C_1$-$C_{12}$ alkyl) moieties, hydroxy or methoxy are available from Aldrich Chemical Company or The Dow Chemical Company. The bis(4-

0231053

hydroxyphenyl)-methanes of formula (II) are prepared by known methods, such as reacting two equivalents of the $R_1$, $R_2$ - substituted phenol with one equivalent of formaldehyde and an acid catalyst. The condensation product formed is a symmetrical compound of formula (II).

In another embodiment, the starting substrate material may be quinone methide of formula (IV). Quinone methides of this type can be produced by the method disclosed in U.S. Patent 2,940,988, which teaches oxidation of dihydroxydiphenyl methane using a lead dioxide or lead tetraacetate oxidant to produce a stable free radical which can be reduced to quinone methide. Preferred sources for a quinone methide of formula (IV) involve 1) production by electrocatalytic oxidation of bis(4-hydroxyphenyl)methane of formula (II) using DDQ of formula (III) and 2) production of quinone methide by electrolytic oxidation of bis(4-hydroxyphenyl)methane, respectively. Should the electrocatalytic process be used to prepare a quinone methide starting material, it is not necessary to separate the quinone methide from the DDQ and $DDQH_2$ prior to its use in the present process. Rather, those impurities are active ingredients in the present electrocatalytic process. Should a pure quinone methide starting material be desired, then the electrolytic process would probably be preferred for economic reasons. For example, the starting materials for the electrocatalytic process [bis(4-hydroxyphenyl)-methane of formula (II) and the oxidant of formula (III)] are dissolved in the supporting electrolyte solution in the anode compartment and stirred during application of a constant voltage vs. SCE of between 0.65 to 1.40 V

and most preferably of 0.75 to 0.95 V vs. SCE. The electrolysis is allowed to proceed until integration of the cell current indicates a passage of 2 Faradays of charge per mole of staring reactant material. At that time, the cell circuit is disconnected and the quinone methide isolated and separated. This may be accomplished by a two step process in which the solvent solution is removed from the quinone methide, $DDQH_2$ and DDQ by evaporation. The quinone methide may, then, be separated from the $DDQH_2$ and DDQ by dissolving it in a solvent which is a non-solvent for $DDQH_2$ or DDQ. For example, quinone methide can be separated from DDQ and $DDQH_2$ by dissolving the quinone methide in methylene chloride, filtering out the still solid DDQ or $DDQH_2$, and, then, re-precipitating the quinone methide.

With any of these embodiments, the starting materials are dissolved in the supporting electrolyte solution in the anode compartment and stirred during application of a constant voltage vs. SCE of between 0.65 V and 1.40 V and most preferably of approximately 0.75 V to 0.95 V vs. SCE. The electrolysis is allowed to come to equilibrium. Dihydroxybenzophenone is produced in yields of 75 to 95 percent and is isolated by evaporating the solvent and filtering off the solid dihydroxybenzophenone as it precipitates out. The dihydroxybenzophenone can be separated from any coprecipitated DDQ by dissolving the dihydroxybenzophenone in acetic acid, filtering out the DDQ, and, then, re-precipitating the dihydroxybenzophenone.

The following examples are illustrative.

Example 1

This example illustrates the preparation of the quinone methide of bis(3,5-dimethyl-4-hydroxyphenyl)-methane. A divided batch electrochemical cell was fitted with platinum working and auxiliary electrodes and a saturated calomel reference electrode (SCE). The cathode (auxiliary) compartment was filled with an electrolyte solution which contained 0.25 M sodium acetate dissolved in one part by volume of acetic acid and four parts of acetonitrile. The anode (working) compartment was charged with one volume of water and then filled with five volumes of the electrolyte solution to which had been added 40 g bis(3,5-dimethyl-4-hydroxyphenyl)methane and 36 g $DDQH_2$ per liter of electrolyte to give a 10 ml solution. The anode was then biased to 0.75 volts vs. SCE. The electrolysis was continued for 148 minutes at which time current integration showed that there had passed 2 Faradays of charge per mole of bis(3,5-dimethyl-4-hydroxyphenyl)-methane in the anode compartment. At that time the cell circuit was disconnected. Gas chromatographic analysis of the anolyte revealed essentially complete conversion of bis(3,5-dimethyl-4-hydroxyphenyl)methane to the corresponding quinone methide.

Example 2

Additional runs were made using the system of Example 1 with solvent/supporting solutions containing 160 mM bis(3,5-dimethyl-4-hydroxyphenyl)methane and $DDQH_2$ form 10 to 100 mole percent relative to the bis(3,5-dimethyl1-4-hydroxyphenyl)methane. Essentially quantitative conversion to the quinone methide was observed in each instance. However, if the oxidation

is allowed to continue past the 2 Faraday/mole stage, conversion of the quinone methide to 3,3',5,5'-tetramethyl-4,4'-dihydroxybenzophenone takes place.

Example 3

This example illustrates the preparation of 3,3',5,5'-tetramethyl-4,4'-dihydroxybenzophenone. A divided batch electrochemical cell was fitted with platinum working and auxiliary electrodes and a saturated calomel reference electrode (SCE). The cathode (auxiliary) compartment was filled with an electrolyte solution which contained 0.25 M sodium acetate dissolved in one part by volume of acetic acid and four parts of acetonitrile. The anode (working) compartment was charged first with one volume of water and then filled with five volumes of the electrolyte solution to which had been added 40 g bis (3,5-dimethyl-4-hydroxyphenyl)methane and 36 g $DDQH_2$ per liter of electrolyte. The anode was then biased to 0.75 volts vs. SCE until equilibrium was reached (overnight, about 16 hours). At that point in time, an 87 to 89 percent yield of 3,3',5,5'-tetramethyl-4,4'-dihydroxybenzophenone was found to have been produced. One hundred percent of the bis(3,5-dimethyl-4-hydroxyphenyl)methane was found to have been consumed.

CLAIMS

1. A process for preparing a dihydroxybenzophenone of the formula

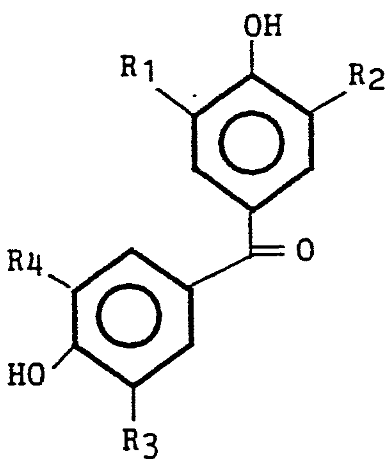

(I)

wherein each $R_1$, $R_2$, $R_3$ and $R_4$ are independently hydrogen, a straight or branched chain $C_1$-$C_{12}$ alkyl moiety, a $C_3$-$C_7$ cyclic alkyl group, halogen, a halo-($C_1$-$C_{12}$ alkyl) moiety, hydroxy, or methoxy groups, which comprises electrocatalytically oxidizing a bis(4-hydroxyphenyl)methane of the formula

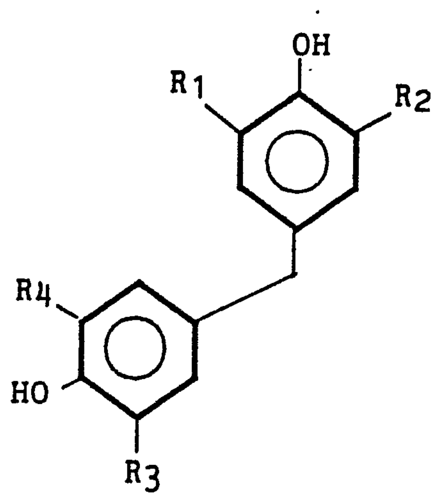

(II)

wherein R1, R2, R3 and R4 are defined as above, with 2,3-dichloro-5,6-dicyano-1,4-benzoquinone of the formula

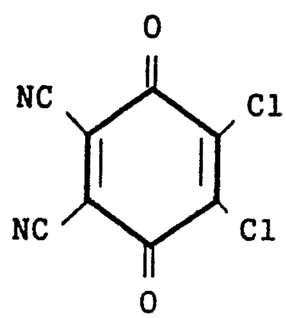

(III)

to produce a quinone methide of the formula

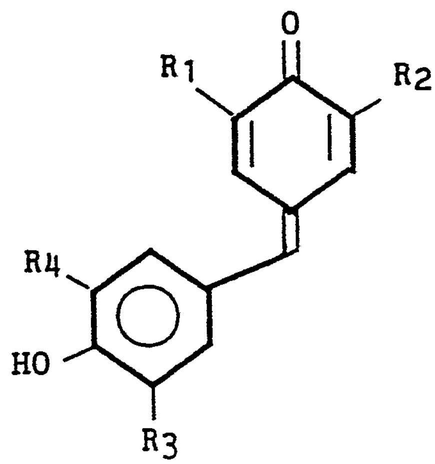

(IV)

wherein $R_1$, $R_2$, $R_3$, and $R_4$ are defined as above, which is further oxidized to provide the compounds of formula (I).

2. A process as claimed in Claim 1, wherein the compound of formula (III) is electrochemically regenerated to its active form after its use in the reaction, and is recycled in the process.

3. A process as claimed in Claim 1 or Claim 2 wherein the compound of formula (III) is present in an amount of from 10 to 100 mole percent relative to said bis(4-hydroxyphenyl)methane of formula (II).

4. A process as claimed in Claim 3, wherein the compound of formula (III) is present in an amount of approximately 10 mole percent relative to said bis(4-hydroxyphenyl)methane of formula (II).

5. A process as claimed in any one of the preceding claims wherein the electrocatalytic oxidation takes place in the presence of a platinum anode at a

voltage of 0.65 to 1.40 V vs. SCE.

6. A process as claimed in any one of the preceding claims, wherein said oxidation takes place in the presence of a platinum anode at a constant voltage of 0.75 to 0.95 V vs. SCE.

7. A process as claimed in any one of the preceding claims, wherein the electrocatalytic oxidation is carried out until there has passed 2 Faradays of charge per mole of bis(4-hydroxyphenyl)-methane of formula (II) to produce a quinone methide of formula (IV).

8. A process as claimed in any one of the preceding claims, wherein said bis(4-hydroxyphenyl)methane of formula (II) is bis(3,5-dimethyl-4-hydroxyphenyl) methane.

9. A process as claimed in any one of the preceding claims, wherein the process is carried out for a period of time sufficient to produce a 75 to 95 percent yield of a dihydroxybenzophenone of formula (I).

10. A process as claimed in any one of the preceding claims, wherein the bis(4-hydroxyphenyl)methane of formula (II) is bis(3,5-dimethyl-4-hydroxyphenyl)methane to provide, as the dihydroxybenzophenone of formula (I), 3,3', 5,5'-tetramethyl-4,4'-dyhydroxybenzophenone.

11. A process as claimed in any one of the preceding claims, wherein the reaction is carried out to equilibrium.

12. A process for preparing a dihydroxybenzophenone of the formula wherein each R1, R2,

R3 and R4 are independently hydrogen, a straight or branched chain C1-C12 alkyl moiety, a C3-C7 cyclic alkyl group, halogen, a halo-(C1-C12 alkyl) moeity, hydroxy, or methoxy groups, which comprises electrocatalytically oxidizing a quinnone methide of the formula (IV) as defined in Claim 1.

13. A process as claimed in any of the preceding claims wherein said oxidant of formula (III) is present in the amount of 10 to 100 mole percent relative to said quinone methide of formula (IV).

0231053

European Patent Office

# EUROPEAN SEARCH REPORT

Application number: EP 87300027.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A2 - 0 012 942 (BASF)<br>* Abstract; page 3, line 9 *<br>-- | 1,5 | C 25 B 3/02<br>//C 07 C 49/83 |
| A | EP - A1 - 0 072 914 (BASF)<br>* Claim 1 *<br>-- | 1 | |
| A | US - A - 3 592 748 (WEHRLI)<br>* Claim 1 *<br>-- | 1 | |
| A | US - A - 4 061 548 (JONES et al.)<br>* Abstract *<br>---- | 1 | |

TECHNICAL FIELDS SEARCHED (Int. Cl.4)

C 25 B

The present search report has been drawn up for all claims

| Place of search | Date of completion of the search | Examiner |
|---|---|---|
| VIENNA | 08-04-1987 | LUX |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document